# EUROPÄISCHE PATENTANMELDUNG

(11) **EP 3 132 788 A1**
(43) Veröffentlichungstag der Anmeldung: **22.02.2017**
(21) Anmeldenummer: 16184110.1
(22) Anmeldetag: 12.08.2016
(51) Int. Cl.: A61K 6/00, A61K 6/02, C04B 41/85, C04B 35/486, C04B 41/50

(54) **EINFÄRBELIQUID**

(30) Priorität: 21.08.2015 DE 102015216056
(71) Anmelder: Dental Direkt GmbH, 32139 Spenge (DE)
(72) Erfinder: Greitens, Uwe, 32139 Spenge (DE); Heilemann, Nadine, 32139 Spenge (DE)
(74) Vertreter: Knoop, Philipp

(57) **Zusammenfassung**

Sinteradditiv (3) zum Hinzufügen zu einem für eine Sinterung vorgesehenen Restaurationsrohkörper (1, 10), insbesondere einem Zirkoniumrestaurationsrohkörper, zur Beeinflussung der Farbanmutung eines Restaurationskörpers (16) in einem gesinterten Zustand, mit einem Medium zur Infiltration poröser Infiltrationsbereiche (6) des Restaurationsrohkörpers (1, 10) und mindestens einem in dem Medium gelösten Stoff, welches eine verbesserte ästhetische Farbanmutung für Zahnrestaurationen bereitstellt, dadurch gekennzeichnet, dass der Stoff zur Steigerung der Transluzenz der Infiltrationsbereiche (6) durch eine Gefügeumwandlung während der Sinterung ausgestaltet ist.

## Beschreibung

Die vorliegende Erfindung betrifft ein Sinteradditiv zum Hinzufügen zu einem für eine Sinterung vorgesehenen Restaurationsrohkörper, insbesondere einem Zirkoniumrestaurationsrohkörper, um die Farbanmutung eines Restaurationskörpers in einem gesinterten Zustand zu beeinflussen, mit einem Medium zur Infiltration poröser Infiltrationsbereiche des Restaurationsrohkörpers und mindestens einem in dem Medium gelösten Stoff.

Weiterhin betrifft die Erfindung eine Verwendung eines derartigen Sinteradditivs zum Hinzufügen zu einem für eine Sinterung vorgesehenen Restaurationsrohkörper, insbesondere einem Zirkoniumrestaurationsrohkörper, um die Farbanmutung eines Restaurationskörpers in einem gesinterten Zustand zu beeinflussen.

Außerdem betrifft die Erfindung einen Restaurationsrohkörper, insbesondere einen Zirkoniumrestaurationsrohkörper, in einem vorgesinterten Zustand, mindestens einen Stoff in einem ersten Bereich enthaltend und den Stoff in einem zweiten Bereich enthaltend und einer ersten Konzentration des Stoffs in dem ersten Bereich und einer zweiten Konzentration des Stoffs in dem zweiten Bereich.

Ferner betrifft die Erfindung einen Restaurationskörper, insbesondere einen Zirkoniumrestaurationskörper, in einem gesinterten Zustand, mit mindestens einem Außenvolumenbereich und mindestens einem an den Außenvolumenbereich angrenzenden Innenvolumenbereich und jeweils einem die Transluzenz des Außenvolumenbereichs kennzeichnenden äußeren Transluzenzwert und einem die Transluzenz des Innenvolumenbereichs kennzeichnenden inneren Transluzenzwert.

Auch betrifft die Erfindung ein Verfahren zur Beeinflussung der Farbanmutung eines Restaurationskörpers, insbesondere eines Zirkoniumrestaurationskörpers, in einem gesinterten Zustand mit einem ersten Schritt, bei dem ein Sinteradditiv einem Restaurationsrohkörper hinzugefügt wird, und einem zweiten Schritt, bei dem der Restaurationsrohkörper mit einem ein Temperatur-Zeitprofil aufweisenden Sinterverfahren gesintert wird.

Zudem betrifft die Erfindung ein Restaurationskörperfinishingkit für eine Beeinflussung der Farbanmutung eines Restaurationskörpers, insbesondere eines Zirkoniumrestaurationskörpers, in einem gesinterten Zustand, aufweisend einen für eine Sinterung vorgesehenen Restaurationsrohkörper, ein Sinteradditiv nach einem der Ansprüche 1 bis 7 und ein Applikationsmittel zur Applikation des Sinteradditivs auf dem Restaurationsrohkörper.

Sinteradditive der eingangs genannten Art werden häufig bei einer vollständigen Sinterung keramischer Zahnrestaurationen zum Färben der Zahnrestaurationen verwendet, um eine Reproduktion einer möglichst natürlichen Farbgebung eines Zahns zu erreichen. Als Zahnrestaurationen kommen beispielsweise monolithische Kronen und Brücken für einen synthetischen Zahnersatz bei einem Patienten in Frage. Solche Zahnrestaurationen bestehen häufig aus vollständig gesintertem Zirkoniumoxid der stöchiometrischen Basiszusammensetzung ZrO₂. Sinteradditive der genannten Art werden in DE 19904522C5 anhand von Metallionen-Lösungen oder Metallkomplex-Lösungen zum Färben der genannten Zahnrestaurationen offenbart. In der Praxis trägt zum Beispiel ein Zahntechniker eine solche Lösung auf eine der genannten Zahnrestaurationen vor ihrer Sinterung auf, um der Zahnrestauration hierdurch eine bestimmte Farbanmutung nach der Sinterung zu verleihen.

Ein Nachteil dieser Lösungen besteht jedoch darin, dass die zu erzielenden Farbergebnisse von einer Vielzahl von Faktoren abhängig sind, wie beispielsweise von der Wandstärke von Restauration, der Tauchzeit beziehungsweise der Häufigkeit des Farbauftrags und von der Erfahrung eines Zahntechnikers. Ein Nachteil besagter Färbelösungen ist daher die oft unzureichende Reproduzierbarkeit der Farbergebnisse. Dazu kommt als weiterer Nachteil die mangelnde Farbschichtstärke. Die Infiltrationstiefe gängiger Färbelösungen ist von der Tauchzeit beziehungsweise von der Häufigkeit des Farbauftrages abhängig. Mit steigender Infiltrationstiefe nimmt auch die Intensität der Farbe zu. Im Schnitt beträgt die Farbschicht etwa bis zu 1 mm, darunter (wie beispielsweise bei massiven Strukturen in Brückengliedern) bleibt das ZrO₂ ungefärbt/weiß. Müssen nun Korrekturen an den Restaurationen vorgenommen werden, kann es passieren, dass die Farbschicht punktuell oder größerflächig entfernt wird. Das Resultat besteht häufig in einer inhomogenen/fleckigen Färbung von Restaurationen.

Ein Restaurationsrohkörper der eingangs genannten Art wird häufig als vorgesinterter Sinterrohling für die Sinterherstellung einer synthetischen Zahnrestauration zum Zahnersatz von beispielsweise Frontzähnen bei einem Patienten verwendet. Ein solcher Restaurationsrohkörper weist typischerweise eine im Wesentlichen homogene Zusammensetzung auf. So ist zum Beispiel bei einem Sinterrohling aus einer Oxidkeramik wie beispielsweise Zirkoniumoxid das häufig verwendete Legierungselement Yttrium homogen über das gesamte Volumen des genannten Rohlings verteilt.

Zum Nachteil ästhetischer Ansprüche vermittelt jedoch eine aus einem herkömmlichen Sinterrohling vollständig gesinterte Zahnrestauration nicht die Farbanmutung schichtartig aufgebauter natürlicher Zähne.

Ein Restaurationskörper der eingangs genannten Art wird häufig als synthetische Zahnrestauration zum Zahnersatz von beispielsweise Frontzähnen bei einem Patienten verwendet. Eine solche Zahnrestauration besteht in der Praxis beispielsweise aus einer gesinterten Oxidkeramik wie beispielsweise Zirkoniumoxid mit der stöchiometrischen Basiszusammensetzung ZrO₂. In Hinblick auf eine möglichst natürliche Farbanmutung weist die Zahnrestauration hierbei zum Beispiel eine monochromatische gelbe Vorfärbung auf.

Zum Nachteil ästhetischer Ansprüche vermitteln vorgefärbte Restaurationskörper jedoch die Farbanmutung eines natürlichen Zahnes nur ungenügend. So ist die Nachbildung der Farbanmutung des schichtartigen Aufbaus eines Zahns mit den genannten Restaurationskörpern nicht möglich. Um einen solchen optischen Effekt zu erreichen, wären weitere Bearbeitungsschritte wie Beispiel das Befestigen zusätzlichen Schichtmaterials notwendig, wodurch sich die Laborherstellung solcher Restaurationskörper nicht nur kostenintensiv sondern auch zeitaufwendig gestaltet. Somit sind herkömmliche Restaurationskörper nur bedingt geeignet, die Farbanmutung natürlicher Zähne wiederzugeben.

Ein Verfahren der eingangs genannten Art besteht oftmals aus dem Auftragen eines Farbmittels auf einen Sinterrohling und/oder eine Zahnersatzkonstruktion, welcher bzw. welche in einem Ofen anschließend zu einem vollständig gesinterten Endprodukt gesintert wird. Als Sinterrohling kommt hierfür beispielsweise ein poröser vorgesinterter Restaurationsrohkörper aus Zirkoniumoxid der stöchiometrischen Basiszusammensetzung ZrO₂ in Frage. Das vollständig gesinterte Endprodukt des genannten Verfahrens ist beispielsweise eine gefärbte Zirkoniumoxid-Zahnrestauration.

Nachteilig an dem genannten Verfahren ist jedoch, dass es ästhetischen Ansprüchen an einen gesinterten Restaurationskörper nur ungenügend Rechnung trägt. Während natürliche Zähne - deren Farbanmutung das Ziel ästhetisierender Maßnahmen ist - einen schichtartigen Aufbau aufweisen, lässt sich die hieraus resultierende Farbanmutung mit dem genannten Verfahren bislang nicht reproduzieren. Somit sind herkömmliche Verfahren nur in eingeschränkter Weise geeignet, eine Zahnrestauration mit natürlicher Farbanmutung herzustellen.

Ein Restaurationskörperfinishingkit der eingangs genannten Art besteht typischerweise aus einem Restaurationsrohkörper, einem Sinteradditiv und einem Mittel zum Auftragen des Sinteradditivs auf den Restaurationsrohkörper. Hierbei ist der Restaurationsrohkörper oftmals eine vorgesinterte poröse Zirkoniumoxid-Zahnrestauration. In der Praxis bearbeitet ein Zahntechniker einen solchen Restaurationsrohkörper beispielsweise mit einem Fräswerkzeug, um dem Restaurationsrohkörper eine für einen Patienten individualisierte Struktur zu verleihen. Ein Sinteradditiv des genannten Restaurationskörperfinishingkits ist in diesem Zusammenhang oftmals ein Färbemittel, welches mit Hilfe beispielsweises eines Pinsels auf den gefrästen Restaurationsrohkörper von einem Zahntechniker aufgetragen wird. Nach dem Auftragen des Färbemittels wird der Restaurationsrohkörper in einem Ofen vollständig gesintert. Unter zahnästhetischen Geschichtspunkten sollten vollständig gesinterte Restaurationskörper zum Beispiel die Farbanmutung des schichtartigen Aufbaus natürlicher Zähne wiedergeben. Diese Farbanmutung lässt sich aber mit Nachteil mit dem genannten Restaurationskörperfinishingkit nicht reproduzieren. Daher schränkt ein herkömmliches Restaurationskörperfinishingkit die ästhetischen Gestaltungsmöglichkeiten eines Zahntechnikers ein.

Der vorliegenden Erfindung liegt daher die Aufgabe zugrunde, ein Sinteradditiv der eingangs genannten Art, eine Verwendung der eingangs genannten Art, einen Restaurationsrohkörper der eingangs genannten Art, einen Restaurationskörper der eingangs genannten Art, ein Verfahren der eingangs genannten Art sowie ein Restaurationskörperfinishingkit der eingangs genannten Art anzugeben, welche unter Umgehung der Nachteile des Standes der Technik eine verbesserte ästhetische Farbanmutung für Zahnrestaurationen bereitstellen.

Erfindungsgemäß wird diese Aufgabe durch ein Sinteradditiv der eingangs genannten Art gelöst, bei dem der Stoff zur Steigerung der Transluzenz der Infiltrationsbereiche durch eine Gefügeumwandlung während der Sinterung ausgestaltet ist. Erfindungsgemäß besteht der Stoff beispielsweise aus Metallionen eines Metallsalzes wie beispielsweise Yttrium(III)nitrat Hexahydrat. So kann das erfindungsgemäße Sinteradditiv zum Beispiel 67 Gewichts.-% Yttrium(III)nitrat Hexahydrat und 33 Gewichts.-% wässrige Salzsäurelösung enthalten. Erfindungsgemäß liegen in diesem Beispiel Yttrium-Ionen in gelöster Form vor. Durch erfindungsgemäße Infiltration poröser Bereiche eines Sinterrohlings mit der genannten Flüssigkeit gelangen zum Beispiel Yttrium-Ionen in Poren des Sinterrohlings, sodass der Sinterrohling in derart infiltrierten Bereichen mit diesen Metallionen angereichert wird. Im Rahmen der Erfindung kann der Sinterrohling beispielsweise ein vorgesinterter Zahnrestaurationsrohkörper der stöchiometrischen Basiszusammensetzung ZrO₂ mit <10 Gewichts.% , vorzugsweise 0,05 - 0,25 Gewichts.-%, Al₂O₃ und ca. 6 Gewichts.-% Y₂O₃ sein. Erfindungsgemäß können auch Magnesium Ionen aus beispielsweise Magnesium Chlorid Hexahydrat als Stoff zur Infiltration verwendet werden. In bevorzugter Ausgestaltung der Erfindung ist die Zusammensetzung des erfindungsgemäßen Sinteradditivs derart auf die Zusammensetzung des Restaurationsrohkörpers abgestimmt ist, dass mit dem Sinteradditiv behandelte Teilvolumina des Restaurationsrohkörpers eine Y₂O₃ Konzentration von etwa 10 Gewichts.% aufweisen. Die genannte erfindungsgemäße Infiltration kann zum Beispiel durch Eintauchen des genannten Sinterrohlings und/oder der Zahnersatzkonstruktion in das erfindungsgemäße Sinteradditiv erreicht werden. Die erfindungsgemäße Anreicherung mit Metallionen bewirkt während einer Sinterung eine Gefügeumwandlung durch beispielsweise Ausscheidung der kubischen Phase von Zirkonium oder einer anderen Phase, was überraschend vorteilhaft für die ästhetische Farbanmutung der gesinterten Zahnrestauration ist. Die erfindungsgemäße Gefügeumwandlung führt zu einer erhöhten Transluzenz infiltrierter Bereiche, sodass bei einem Zahnrestaurationskörper im vollständig gesinterten Zustand die Farbanmutung höhertransluzenten Zahnschmelzes mithilfe des erfindungsgemäßen Sinteradditivs zum Vorteil der Ästhetik reproduziert wird. Zudem ist das erfindungsgemäße Sinteradditiv auch vor dem Hintergrund einer Patientenverträglichkeit vorteilhaft, indem der gelöste Stoff des Sinteradditivs zu den in der zahnmedizinischen Praxis bewährten Legierungselementen von Zirkonium gehören kann, wie zum Beispiel Yttrium oder Magnesium.

Überraschenderweise kann mit dem erfindungsgemäßen Sinteradditiv auch eine Aufhellung infiltrierter Bereiche eines vollständig gesinterten Zahnrestaurationskörpers erreicht werden, was sich besonders vorteilhaft auf die ästhetischen Gestaltungsmöglichkeiten bei der Färbung von zum Beispiel voreingefärbten Zahnrestaurationskörpern auswirkt.

Nachstehender Tabelle 1 sind beispielhafte Zusammensetzungen des erfindungsgemäßen Sinteradditivs zu entnehmen. Die erste Spalte in der Tabelle 1 enthält zum Zweck einer Bezugnahme den vom Erfinder gewählten Kurznamen des erfindungsgemäßen Sinteradditivs hinsichtlich der in den Spalten 2, 3, 4, 5 und 6 angegebenen Zusammensetzung.

**Tabelle 1: Beispielhafte Zusammensetzung des erfindungsgemäßen Sinteradditivs.**

| Kurzname des erfindungsgemäßen Sinteradditivs | Beispielhafte Zusammensetzung des erfindungsgemäßen Sinteradditivs aus | | | |
|---|---|---|---|---|
| | Yttrium(III)-nitrat Hexahydrat [Gew.-%] | Magnesium-chlorid Hexahydrat [Gew.-%] | Zirkon(IV) Hydrat [Gew.-%] | wässrige Salzsäurelösung |
| Y67 | 67 | 0 | 0 | 33 |
| Y50Mg15 | 50 | 15 | 0 | 35 |
| Y50Z15 | 50 | 0 | 15 | 35 |
| Z50 | 0 | 0 | 50 | 50 |
| Mg60 | 0 | 60 | 0 | 40 |
| Mg70 | 0 | 70 | 0 | 30 |
| Y5M60 | 5 | 60 | 0 | 35 |

In vorteilhafter Ausgestaltung des erfindungsgemäßen Sinteradditivs ist die Gefügeumwandlung eine Kornvergröberung. Die erfindungsgemäße Kornvergröberung kann zum Beispiel aus dem Wachstum vorhandener Körner und/oder der Bildung neuer Körner durch zum Beispiel Ausscheidungsreaktionen bestehen. Ein durch die Kornvergröberung umgewandeltes Gefüge ist überraschenderweise durch eine Transluzenz gekennzeichnet, welche gegenüber dem übrigen Gefüge eines vollständig gesinterten Zahnrestaurationskörpers erhöht ist. Zum Vorteil der Zahnästhetik verleiht die erfindungsgemäße Kornvergröberung einem Zahnrestaurationskörper somit eine Farbanmutung, die in Analogie zu natürlichen Zähnen eine höhertransluzente Oberfläche mit einem darunterliegenden Volumen erhöhter Opazität aufweist.

In einer weiteren vorteilhaften Ausgestaltung des erfindungsgemäßen Sinteradditivs stellt eine Ausscheidung der kubischen Phase von Zirkonium die Kornvergröberung im Wesentlichen bereit. Erfindungsgemäß wird hierbei die Kornvergröberung über eine Ausscheidungsreaktion gesteuert, welche als Endprodukt oder metastabiles Zwischenprodukt die kubische Phase von Zirkonium hat. Der Phasenanteil und somit das Ausmaß der Kornvergröberung, welche erfindungsgemäß für die ästhetische Anmutung eines Zahnrestaurationskörpers vorteilhaft ist, lässt sich daher neben der Temperatur auch über die Zusammensetzung eines Zirkonium-Zahnrestaurationsrohkörper steuern, zum Beispiel durch eine Variation des Yttrium-Gehalts. Anhand der Wahl einer geeigneten Legierungszusammensetzung steht somit einem Zahntechniker zum Vorteil der Zahnästhetik überraschenderweise eine zusätzliche Einflussgröße zur Verfügung, nämlich die Zusammensetzung, um die Farbanmutung eines vollständig gesinterten Zahnrestaurationskörpers erfindungsgemäß zu beeinflussen.

In noch einer weiteren Ausgestaltung des erfindungsgemäßen Sinteradditivs ist der Stoff ein chemisches Element aus der Gruppe der die kubische Phase stabilisierenden Elemente. Ein erfindungsgemäßer Stoff kann somit Magnesium, Calcium, Yttrium oder Cer verwendet werden, da von diesen chemischen Elementen bekannt ist, dass sie die kubische Phase von Zirkonium stabilisieren. Mit dem Begriff "chemisches Element" sind im Rahmen der Erfindung auch die aus Metallsalzen oder Metalloxiden stammenden Ionen gemeint. Ist der erfindungsgemäße Stoff eines der genannten Elemente, begünstigt dies die Ausscheidung der kubischen Phase von Zirkonium, was sich überraschenderweise positiv auf die ästhetische Farbanmutung eines mit dem erfindungsgemäßen Sinteradditiv infiltrierten Zahnrestaurationskörper der beispielsweise stöchiometrischen Basiszusammensetzung ZrO₂ mit <10 Gewichts.% , vorzugsweise 6 Gewichts.-% Y₂O₃ im vollständig gesinterten Zustand auswirkt.

In vorteilhafter Ausgestaltung des erfindungsgemäßen Sinteradditivs ist das Medium flüssig. Als flüssiges Medium kann im Rahmen der Erfindung beispielsweise Wasser verwendet werden. Der erfindungsgemäß flüssige Zustand des Medium erleichtert zum Vorteil des Hinzufügens des Sinteradditivs dieser Erfindung zu einem porösen vorgesinterten Restaurationsrohkörper die Infiltration des Restaurationsrohkörpers, da zum Beispiel Kapillarkräfte für die Infiltration zu Verfügung stehen.

Bei einer weiteren vorteilhaften Ausgestaltung des erfindungsgemäßen Sinteradditivs enthält das Medium eine Säure, insbesondere eine anorganische Säure, zur Erhöhung der Löslichkeit des Stoffs. Um beispielsweise die Löslichkeit von Yttrium-Ionen, welche zum Beispiel durch das Metallsalz Yttrium(III)nitrat Hexahydrat einer wässrigen Lösung zugegeben wurden, zu erhöhen, kann man erfindungsgemäß eine wässrige Salzsäure verwenden. Zum Vorteil der ästhetischen Gestaltung eines Zahnrestaurationskörpers können durch die erfindungsgemäße Zugabe einer Säure die für eine Färbung der Zahnrestaurationskörper erforderlichen Stoffmengen in einem breiten Bereich eingestellt werden. So können in einer wässrigen Lösung mithilfe der erfindungsgemäßen Zugabe beispielsweise etwa 67 Gewichts-% Yttrium(III)nitrat Hexahydrat oder zum Beispiel 70 Gewichts.-% Magnesiumchlorid Hexahydrat mithilfe einer wässrigen Salzsäurelösung gelöst werden.

In vorteilhafter Ausgestaltung des erfindungsgemäßen Sinteradditivs enthält das Medium ein Viskositätsmittel zur Erhöhung der Viskosität des Mediums. Einer wässrigen Lösung, in der erfindungsgemäß beispielsweise Yttrium-Ionen gelöst sind, kann als erfindungsgemäßes Viskositätsmittel beispielsweise ein Stoff aus den Gruppen Polyglykole, Polysaccharide, Polyethylenglykole zugegeben werden. Überraschenderweise erhöht dies die Viskosität der Lösung, sodass sich zum Vorteil der Färbung eines Restaurationskörpers mit Hilfe des erfindungsgemäßen Sinteradditivs die Tiefe und Homogenität der Infiltration der Lösung in dem Restaurationskörpers einstellen lässt.

In einer weiteren vorteilhaften Ausgestaltung des erfindungsgemäßen Sinteradditivs enthält das Medium mindestens ein Farbmittel zur Markierung von Applikationsbereichen des Sinteradditivs auf dem Restaurationsrohkörper und das Mittel ist durch die Sinterung entfernbar. Als erfindungsgemäßes Farbmittel können organische Pigmente verwendet werden, welche bei den üblicherweise sehr hohen Sintertemperaturen von beispielsweise 1400°C rückstandsfrei verbrennen. Zum Beispiel können im Rahmen der Erfindung Pigmente bestehend aus synthetischen Azo- oder Triphenylmethanfarbstoffen verwendet werden. Die erfindungsgemäßen Applikationsbereiche des Sinteradditivs auf einem Restaurationsrohkörper, welcher in einem porösen vorgesinterten Zustand vorliegt, können zum Beispiel die Kanten des Restaurationsrohkörper sein, um dort eine lokale Veränderung der Farbanmutung mithilfe des erfindungsgemäßen Sinteradditivs zu erzielen. Das erfindungsgemäße Färbemittel erleichtert es dem Zahntechniker vorteilhaft, die Bereiche eines Restaurationsrohkörpers zu identifizieren, auf welche das Sinteradditiv bereits mit Hilfe eines zum Beispiel Pinsels aufgetragen wurde.

In Bezug auf einen Restaurationsrohkörper der eingangs genannten Art wird die Aufgabe überraschend dadurch gelöst, dass die erste Konzentration höher als die zweite Konzentration ist und dass der Stoff zur Steigerung der Transluzenz des ersten Bereichs durch eine Gefügeumwandlung während einer Sinterung des Restaurationsrohkörpers ausgestaltet ist. Erfindungsgemäß besteht ein Restaurationsrohkörper beispielsweise aus zwei Bereichen verdichteten Pulvers unterschiedlicher Zusammensetzung mit der stöchiometrischen Basiszusammensetzung ZrO₂. Das Zusammenfügen der genannten Bereiche kann zum Beispiel durch einen kaltisostatischen Pressvorgang erreicht werden. Erfindungsgemäß unterscheidet sich die genannte Zusammensetzung hierbei zum Beispiel hinsichtlich eines Gehalts an Y₂O₃, sodass der eine Bereiche gegenüber dem anderen Bereich einen höheren Gehalt an Y₂O₃ aufweist. Überraschenderweise führt die durch die Anwesenheit von Y₂O₃ unterstützte Ausscheidung der kubischen Phase von Zirkonium während einer Sinterung des erfindungsgemäßen Restaurationsrohkörpers zu einer erhöhten Transluzenz des erfindungsgemäßen Bereichs mit höherem Gehalt an Y₂O₃. Dies beeinflusst die Farbanmutung einer aus dem erfindungsgemäßen Restaurationsrohkörper durch eine Sinterung hergestellten Zahnrestauration positiv, da die erfindungsgemäß erhöhte Transluzenz die Farbanmutung eines natürlichen Zahnes reproduziert.

In Bezug auf einen Restaurationskörper der eingangs genannten Art wird die Aufgabe überraschend dadurch gelöst, dass der äußere Transluzenzwert größer als der innere Transluzenzwert ist. Ein erfindungsgemäßer Restaurationskörper kann zum Beispiel aus einer vollständig gesinterten Keramik der stöchiometrischen Basiszusammensetzung ZrO₂ mit <10 Gewichts.% , vorzugsweise 6 Gewichts.-% Y₂O₃ bestehen. In vorteilhafter Weise gibt der erfindungsgemäße Restaurationskörper den schichtartigen Aufbau vieler natürlicher Zähne wieder, welche aus einer höhertransluzenten zahnschmelzhaltigen Oberflächenschicht und einem unter dieser Oberflächenschicht liegenden dentinhaltigen Volumen mit erhöhter Opazität bestehen. Ästhetisch besonders ansprechend ist ein erfindungsgemäßer Restaurationskörper mit einer monochromatischen Vorfärbung, sodass mit dem erfindungsgemäße Restaurationskörper die Farbanmutung von farbintensiven Dentin mit einer darüberlegenden transluzenten Zahnschmelzschicht reproduziert wird.

In einer weiteren vorteilhaften Ausgestaltung des erfindungsgemäßen Restaurationskörpers ist der Außenvolumenbereich an mindestens einer Kante des Restaurationskörpers angeordnet. Im Rahmen der Erfindung kann der Außenvolumenbereich zum Beispiel die Schneidekante eines durch den erfindungsgemäßen Restaurationskörper reproduzierten Schneidezahns sein. Hierdurch wird zum Vorteil einer natürlichen Farbanmutung des erfindungsgemäßen Restaurationskörpers erreicht, dass die Schneidezahnkante in Analogie zu einem natürlichen Schneidezahn eine höhere Transluzenz aufweist als Bereiche unterhalb der Schneidezahnkante.

In Bezug auf ein Verfahren der eingangs genannten Art wird die Aufgabe überraschenderweise dadurch gelöst, dass das Sinteradditiv nach einem der Ansprüche 1 bis 7 ausgestaltet ist. Das erfindungsgemäße Verfahren besteht zum Beispiel aus dem Eintauchen eines Restaurationsrohkörpers in ein erfindungsgemäßes Sinteradditiv und einer hieran anschließenden vollständigen Sinterung des Restaurationsrohkörpers in beispielsweise einem Ofen. Der Restaurationsrohkörpers kann hierbei beispielsweise eine vorgesinterte poröse Dentalkeramik der stöchiometrischen Basiszusammensetzung ZrO₂ mit <10 Gewichts.% , vorzugsweise 6 Gewichts.-% Y₂O₃ sein, welche nach einer vollständigen Sinterung als Restaurationskörper zum beispielsweise synthetischen Ersatz für einen Frontzahn bei einem Patienten dient. Das erfindungsgemäße Sinteradditiv kann in diesem Zusammenhang beispielsweise eine Mischung aus 50 Gewichts.-% Yttrium(III)nitrat Hexahydrat und 15 Gewichts.-% Magnesiumchlorid Hexahydrat 35 Gewichts.-% einer wässrigen Salzsäurelösung enthalten. Die erfindungsgemäße Sinterung des Restaurationsrohkörpers kann zum Beispiel mit dem folgenden Zeit-Temperatur-Profil durchgeführt werden: Aufheizen auf 900°C bei einer Aufheizrate von 8°C/Minute, Halten bei 900°C für 30 Minuten, Aufheizen auf 1450°C bei einer Aufheizrate von 200°C/Minute, Halten bei 1450°C für 120 Minuten, ungeregeltes Abkühlen auf Raumtemperatur. Überaschenderweise gelingt es mithilfe des erfindungsgemäßen Verfahrens, die Farbanmutung des vollständig gesinterten Restaurationskörpers zum Vorteil der Zahnästhetik zu gestalten. So entspricht die durch das erfindungsgemäße Verfahren beeinflusste Farbanmutung der Farbanmutung eines schichtartig aufgebauten Zahns mit einer höhertransluzenten zahnschmelzhaltigen Oberflächenschicht und einem unter dieser Oberflächenschicht liegenden dentinhaltigen Volumen mit erhöhter Opazität.

In einer weiteren bevorzugten Ausgestaltung des erfindungsgemäßen Verfahrens weist das Temperatur-Zeitprofil mindestens ein Temperaturplateau mit einer Plateautemperatur und einer Plateauzeitspanne auf, wobei die Plateautemperatur gleich oder größer als eine für eine Gefügeumwandlung in von dem Sinteradditiv infiltrierten Infiltrationsbereichen des Restaurationsrohkörpers erforderlichen Umwandlungstemperatur ist und die Plateauzeitspanne größer als eine für einen Beginn der Gefügeumwandlung erforderliche Umwandlungszeit ist. Ein erfindungsgemäßes Temperaturplateau kann beispielsweise durch eine Temperatur von 1450°C und eine Zeitspanne von 120 Minuten gekennzeichnet sein. Der einem solchen Temperaturplateau ausgesetzte Restaurationsrohkörper ist zum Beispiel eine vorgesinterte poröse Dentalkeramik der stöchiometrischen Basiszusammensetzung ZrO₂ mit <10 Gewichts.% , vorzugsweise 6 Gewichts.-% Y₂O₃. Das erfindungsgemäße Sinteradditiv kann in diesem Zusammenhang eine Mischung aus 50 Gewichts.-% Yttrium(III)nitrat Hexahydrat und 15 Gewichts.-% Magnesiumchlorid Hexahydrat und 35 Gewichts.-% einer wässrigen Salzsäurelösung enthalten. Das als Beispiel genannte erfindungsgemäße Temperaturplateau ist überraschenderweise hinsichtlich seiner Temperatur (1450°C) und Zeitdauer (120 Minuten) ausreichend groß, um eine Ausscheidung der kubischen Phase von Zirkonium in der von dem Sinteradditiv beispielsweise infiltrieren Oberfläche des Restaurationsrohkörpers zu erreichen. Zum Vorteil einer ästhetischen Gestaltung der Farbanmutung des Restaurationskörpers im vollständig gesinterten Zustand sind die infiltrierten Bereiche hierbei höhertransluzent gestaltet, sodass vorteilhaft die Farbanmutung von transluzentem Zahnschmelz mithilfe des erfindungsgemäßen Verfahrens hervorgerufen wird.

Bei einer weiteren bevorzugten Ausgestaltung des erfindungsmäßen Verfahrens wird das Sinteradditiv auf mindestens einer Kante des Restaurationsrohkörpers aufgetragen. Im Rahmen der Erfindung kann die Kante zum Beispiel die durch einen vorgesinterten Zahnrestaurationsrohkörper der stöchiometrischen Basiszusammensetzung ZrO₂ mit ca. 0,05 - 0,25, Gewichts.-% Al₂O₃ und ca. 6 Gewichts.-% Y₂O₃ reproduzierte Schneidekante eines natürlichen Zahns sein. Das erfindungsgemäße Auftragen des erfindungsgemäßen Sinteradditivs auf eine solche Kante kann zum Beispiel mithilfe eines Pinsels erfolgen. Das erfindungsgemäße Sinteradditiv kann hierbei in der Zusammensetzung 60 Gewichts.-% Magnesiumchlorid Hexahydrat und 40 Gewichts.-% einer wässrigen Salzsäurelösung vorliegen. Mithilfe des erfindungsgemäßen Verfahrens wird zum Vorteil einer natürlichen Farbanmutung des Restaurationskörpers im vollständig gesinterten Zustand erreicht, dass die Schneidezahnkante in Analogie zu einem natürlichen Schneidezahn höhertransluzent ausgestaltet ist.

In Bezug auf ein Restaurationskörperfinishingkit wird die Aufgabe gelöst durch ein Restaurationskörperfinishingkit für eine Beeinflussung der Farbanmutung eines Restaurationskörpers, insbesondere eines Zirkoniumrestaurationskörpers, in einem gesinterten Zustand, aufweisend einen für eine Sinterung vorgesehenen Restaurationsrohkörper, ein Sinteradditiv nach einem der Ansprüche 1 bis 7 und ein Applikationsmittel zur Applikation des Sinteradditivs auf dem Restaurationsrohkörper. Der erfindungsgemäße Zirkoniumrestaurationsrohkörper kann beispielsweise die stöchiometrische Basiszusammensetzung ZrO₂ mit <10, insbesondere ca. 0,05 - 0,25, Gewichts.-% Al₂O₃ und ca. 6 Gewichts.-% Y₂O₃ aufweisen und in einem vorgesinterten porösen sowie vorgefärbten Zustand vorliegen. Das erfindungsgemäße Sinteradditiv kann zum Beispiel 67 Gewichts.-% Yttrium(III)nitrat Hexahydrat und 33 Gewichts.-% einer wässrigen Salzsäurelösung sowie organische Farbpigmente, bestehend beispielsweise aus synthetische Azo- oder Triphenylmethanfarbstoffe, und Polyethylenglykol enthalten. Die erfindungsgemäßen Farbpigmente markieren die Bereiche auf dem Zirkoniumrestaurationsrohkörper, auf die bereits das erfindungsgemäße Sinteradditiv aufgetragen wurde. Die erfindungsgemäßen Farbpigmente verbrennen rückstandsfrei mit Vorteil während einer Sinterung des Zirkoniumrestaurationsrohkörpers. Die erfindungsgemäße Zugabe von beispielsweise Polyethylenglykol mit einer Molmasse von 100 g/mol erhöht die Viskosität der genannten wässrigen Salzsäurelösung, sodass sich das Infiltrationsverhalten dieser Lösung in dem Zirkoniumrestaurationsrohkörper vorteilhaft einstellen lässt.

Als erfindungsgemäße Mittel zum vorteilhaften Auftragen des Sinteradditivs enthält das erfindungsgemäße Restaurationskörperfinishingkit einen Pinsel. Das erfindungsgemäße Restaurationskörperfinishingkit gewährleistet überaschsenderweise mit Vorteil eine Farbanmutung eines vollständig gesinterten Zahnrestaurationsköpers, welcher die Farbanmutung eines natürlichen Zahns mit farbintensivem Dentin und höhertransluzentem Zahnschmelz reproduziert.

Die Erfindung wird in einer bevorzugten Ausführungsform unter Bezugnahme auf eine Zeichnung beispielhaft beschrieben, wobei weitere vorteilhafte Einzelheiten den Figuren der Zeichnung zu entnehmen sind.

Funktionsmäßig gleiche Teile sind dabei mit denselben Bezugszeichen versehen.

Die Figuren der Zeichnung zeigen im Einzelnen:
- Figur 1a:: einen erfindungsgemäßen Zahnrestaurationsrohkörper in Seitenansicht in einem vorgesinterten porösen Zustand;
- Figur 1b:: den erfindungsgemäßen Zahnrestaurationsrohkörper aus Figur 1a in Seitenansicht im mit dem erfindungsgemäßen Sinteradditiv infiltrierten Zustand sowie einen Pinsel und einen das erfindungsgemäße Sinteradditiv bevorratenden Behälter;
- Figur 1c:: einen erfindungsgemäßen Restaurationskörper in Seitenansicht, welcher anhand des Zahnrestaurationsrohkörper aus Figur 1b gesintert wurde, mit schematisch dargestelltem Gefüge; und
- Figur 2:: einen weiteren erfindungsgemäßen Zahnrestaurationsrohkörper in Seitenansicht in einem vorgesinterten porösen und unbearbeiteten Zustand mit einer gestrichelten Zahnkonturlinie und einer durchgezogenen Linie zur Markierung von Bereichen unterschiedlicher chemischer Zusammensetzung.

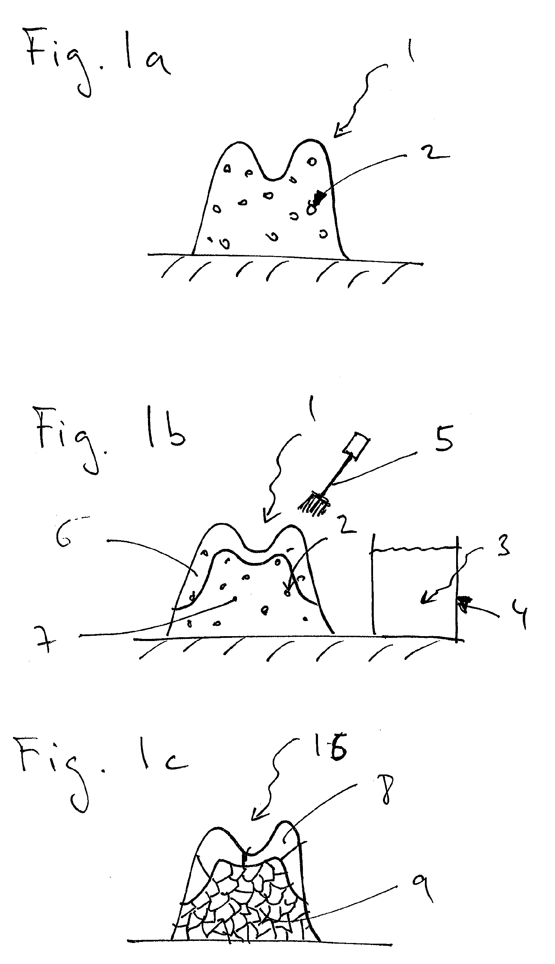

Figur 1a zeigt einen schematisch dargestellten erfindungsgemäßen Zahnrestaurationsrohkörper 1 einer Zirkoniumkeramik der stöchiometrischen Basiszusammensetzung ZrO₂ mit etwa 0,25 Gewichts.-% Al₂O₃, 5 Gewichts.-% Y₂O₃ und weniger als 0,15 Gewichts.-% anderer Oxide in einem vorgesinterten Zustand mit schematisch dargestellten Poren 2 zu entnehmen. Dem Zahnrestaurationskörper 1 wurde eine Zahnstruktur in einem nicht dargestellten Bearbeitungsschritt durch Fräsen verliehen.

Figur 1b zeigt den erfindungsgemäßen Zahnrestaurationsrohkörper 1 aus Figur 1a nachdem ein erfindungsgemäßes Sinteradditiv 3 der Zusammensetzung Y50Mg15 gemäß Tabelle 1 aus einem Behälter 4 mithilfe eines Pinsels 5 auf den Zahnrestaurationsrohkörper 1 aufgetragen wurde. Auf diese Weise wurde der Zahnrestaurationsrohkörper 1 über die Poren 2 in einem Außenbereich 6 gegenüber einem Innenbereich 7 mit dem Sinteradditiv 3 infiltriert.

Figur 1c zeigt einen erfindungsgemäßen Zahnrestaurationskörper 16, welcher durch eine Sinterung bei 1450°C für 2 Stunden in einem Ofen anhand des Zahnrestaurationsrohkörpers 1 aus Figur 1b gesintert wurde. Der Zahnrestaurationskörper 16 hat in einem Außenbereich 8 ein gegenüber einem Innenbereich 9 gröberes Korn. Der grobkörnige Außenbereich 8 stimmt mit dem durch das erfindungsgemäße Sinteradditiv 3 infiltrierten Außenbereich 6 des vorgesinterten Zahnrestaurationsrohkörper 1 aus Figur 1b überein. Der Außenbereich 8 ist hierbei erfindungsgemäß höhertransluzent als der Innenbereich 9, wodurch mit dem Zahnrestaurationsrohkörper 16 erfindungsgemäß die Farbanmutung eines natürlichen Zahns reproduziert wird.

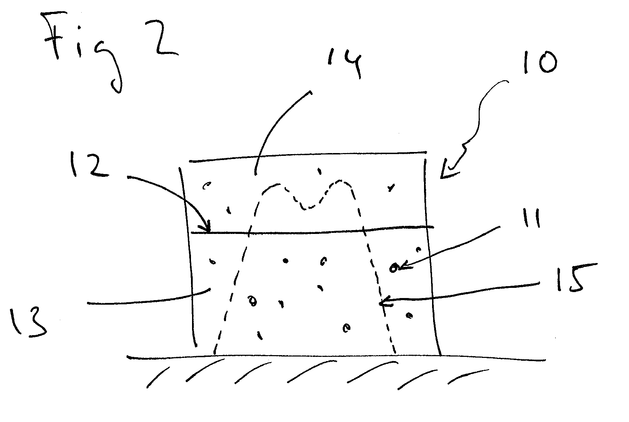

Figur 2 zeigt einen weiteren erfindungsgemäßen Zahnrestaurationsrohkörper 10 in einem vorgesinterten porösen Zustand mit Poren 11. Der Zahnrestaurationsrohkörper 10 hat die stöchiometrischen Basiszusammensetzung ZrO₂. Eine durgezogene Linie 12 des Zahnrestaurationsrohkörpers 10 kennzeichnet eine Grenze zwischen einem yttriumoxidarmen Bereich 13 mit 3 Gewichts.-% Yttriumoxid und einem yttriumoxidreichen Bereich 14 mit 10 Gewichts.-% Yttriumoxid. Eine gestichelte Linie 15 dient als Markierung, anhand derer dem Zahnrestaurationsrohkörper 10 mithilfe eines Fräswerkzeugs die Form eines Zahnes verliehen wird. Erfindungsgemäß führt eine Sinterung des Zahnrestaurationsrohkörpers 10 zu einer Gefügeumwandlung im Bereich 14, welche aufgrund einer Kornvergröberung zu einer im Vergleich zum Bereich 13 höheren Transluzenz des Bereichs 14 führt.

Anhand einer bevorzugten Ausführungsform des erfindungsgemäßen Sinteradditivs ist in nachstehender Tabelle 2 eine qualitative experimentelle Bewertung der Transluzenz und der Helligkeit gesinterter Dentalkeramikplättchen der stöchiometrischen Zusammensetzung ZrO₂ mit etwa 0,25 Gewichts.-% Al₂O₃, 5 Gewichts.-% Y₂O₃ und weniger als 0,15 Gewichts.-% anderer Oxide zu entnehmen, welche für das Experiment zunächst beispielhaft in das erfindungsgemäße Sinteradditiv mit verschiedenen Zusammensetzungen gemäß Tabelle 1 für 20 s, 30 s, 1 Minute oder 2 Minuten getaucht wurden.

**Tabelle 2: Qualitative Bewertung von Transluzenz und Helligkeit nach dem Auftragen des erfindungsgemäßen Sinteradditivs in einer bevorzugten Ausführungsform auf weiße und gelbliche Dentalkeramikplättchen. Weitere Angaben sind dem Text zu entnehmen.**

| Kurzname des erfindungsgemäßen Sinteradditivs | Material-vorfärbung | Transluzenz | Helligkeit |
|---|---|---|---|
| 00 | weiß | ○ | ○○○○ |
| Y67 | | ○○○ | ○○○ |
| 00 | gelblich | ●● | ● |
| Y67 | | ○ | ○○ |
| Y50Mg15 | | ○○ | ○○ |
| Mg60 | | ○ | ○○○ |
| Mg70 | | ● | ○○ |
| Y5M60 | | ○○ | ○○○ |

Die Dentalkeramikplättchen hatten vor dem Eintauchen entweder eine weiße oder eine gelbliche Farbanmutung. Die Dicke der Dentalkeramikplättchen betrug etwa 1 mm, was in der Größenordnung der Dicke von natürlichem Zahnschmelz liegt.

Nach dem Eintauchen der Dentalkeramikplättchen wurden sie bei einer Temperatur von 80°C für 30 Minuten getrocknet. Auf die Trocknung folgte eine Sinterung in einem Ofen des Type Mihm Vogt bei einer Temperatur von 1450°C für zwei Stunden. In analogerweise wurden nicht eingetauchte Referenzplättchen gesintert, deren Transluzenz und Helligkeitswerte folglich aus Referenz dienen. Der Kurzname 00 kennzeichnet dies in Tabelle 2.

Überraschenderweise war die resultierende Farbanmutung nach der Sinterung der Dentalkeramikplättchen von der Tauchzeit unabhängig.

In Tabelle 2 wird das Symbol ○ zu Bezeichnung einer transluzent beziehungsweise hell erscheinenden Farbanmutung verwendet, während das Symbol ● zur Bezeichnung einer opak beziehungsweise dunkel erscheinenden Farbanmutung verwendet wird. Eine höhere Anzahl des genannten Symbols kennzeichnet eine entsprechend zunehmende Tendenz. Beispielsweise ist ein Farbanmutung mit einer Transluzenz von ○○○ deutlich tranluzenter als eine Farbanmutung mit einer Transluzenz von o. In analogerweise ist eine Farbanmutung mit einer Helligkeit von ○○○ deutlich heller als eine Farbanmutung mit einer Helligkeit von o.

Tabelle 2 ist exemplarisch zu entnehmen, dass das erfindungsgemäße Sinteradditiv eine Erhöhung der Transluzenz und eine Erhöhung der Helligkeit bereitstellt.

### BEZUGSZEICHENLISTE

- 1: Zahnrestaurationsrohkörper
- 2: Pore
- 3: Sinteradditiv
- 4: Behälter
- 5: Pinsel
- 6: Außenbereich
- 7: Innenbereich
- 8: Außenbereich
- 9: Innenbereich
- 10: Zahnrestaurationsrohkörper
- 11: Pore
- 12: Grenze
- 13: Bereich
- 14: Bereich
- 15: Linie
- 16: Zahnrestaurationskörper

## Patentansprüche

1. Sinteradditiv (3) zum Hinzufügen zu einem für eine Sinterung vorgesehenen Restaurationsrohkörper (1, 10), insbesondere einem Zirkoniumrestaurationsrohkörper, um die Farbanmutung eines Restaurationskörpers (16) in einem gesinterten Zustand zu beeinflussen, mit einem Medium zur Infiltration poröser Infiltrationsbereiche (6) des Restaurationsrohkörpers (1, 16) und mindestens einem in dem Medium gelösten Stoff, **dadurch gekennzeichnet, dass** der Stoff zur Steigerung der Transluzenz der Infiltrationsbereiche (6) durch eine Gefügeumwandlung während der Sinterung ausgestaltet ist.

2. Sinteradditiv (3) nach Anspruch 1, **dadurch gekennzeichnet, dass** der Stoff Zirkonium-Kationen vorzugsweise in Kombination mit Yttrium-Kationen aufweist.

3. Sinteradditiv (3) nach Anspruch 1 oder 2, **dadurch gekennzeichnet, dass** die Gefügeumwandlung eine Kornvergröberung ist.

4. Sinteradditiv (3) nach Anspruch 3, **dadurch gekennzeichnet, dass** eine Ausscheidung der kubischen Phase von Zirkonium die Kornvergröberung im Wesentlichen bereitstellt.

5. Sinteradditiv (3) nach Anspruch 4, **dadurch gekennzeichnet, dass** der Stoff ein chemisches Element aus der Gruppe der die kubische Phase stabilisierenden Elemente ist.

6. Sinteradditiv (3) nach einem der Ansprüche 1 bis 5, **dadurch gekennzeichnet, dass** das Medium flüssig ist.

7. Sinteradditiv (3) nach Anspruch 6, **dadurch gekennzeichnet, dass** das Medium eine Säure, insbesondere eine anorganische Säure zur Erhöhung der Löslichkeit des Stoffs und/oder ein Viskositätsmittel zur Erhöhung der Viskosität des Mediums und/oder ein Farbmittel zur Markierung von Applikationsbereichen des Sinteradditivs auf dem Restaurationsrohkörper enthält, wobei das Farbmittel durch die Sinterung entfernbar ist.

8. Verwendung des Sinteradditivs (3) nach einem der Ansprüche 1 bis 7 zum Hinzufügen zu einem für eine Sinterung vorgesehenen Restaurationsrohkörper (1, 10), insbesondere einem Zirkoniumrestaurationsrohkörper, um die Farbanmutung eines Restaurationskörpers (16) in einem gesinterten Zustand zu beeinflussen.

9. Restaurationsrohkörper (10), insbesondere ein Zirkoniumrestaurationsrohkörper, in einem vorgesinterten Zustand, mindestens einen Stoff in einem ersten Bereich (12) enthaltend und den Stoff in einem zweiten Bereich (13) enthaltend und einer ersten Konzentration des Stoffs in dem ersten Bereich (12) und einer zweiten Konzentration des Stoffs in dem zweiten Bereich (13), **dadurch gekennzeichnet, dass** die erste Konzentration höher als die zweite Konzentration ist und dass der Stoff zur Steigerung der Transluzenz des ersten Bereichs (12) durch eine Gefügeumwandlung während einer Sinterung des Restaurationsrohkörper (10) ausgestaltet ist.

10. Restaurationskörper (16), insbesondere ein Zirkoniumrestaurationskörper, in einem gesinterten Zustand, mit mindestens einem Außenvolumenbereich (8) und mindestens einem an den Außenvolumenbereich (8) angrenzenden Innenvolumenbereich (9) und jeweils einem die Transluzenz des Außenvolumenbereichs (8) kennzeichnenden äußeren Transluzenzwert und einem die Transluzenz des Innenvolumenbereichs (9) kennzeichnenden inneren Transluzenzwert, **dadurch gekennzeichnet, dass** der äußere Transluzenzwert größer als der innere Transluzenzwert ist.

11. Restaurationskörper (16) nach Anspruch 10, **dadurch gekennzeichnet, dass** der Außenvolumenbereich (8) an mindestens einer Kante des Restaurationskörpers angeordnet ist.

12. Verfahren zur Beeinflussung der Farbanmutung eines Restaurationskörpers (16), insbesondere eines Zirkoniumrestaurationskörpers, in einem gesinterten Zustand, mit einem ersten Schritt, bei dem ein Sinteradditiv (3) einem Restaurationsrohkörper (1, 10) hinzugefügt wird, und einem zweiten Schritt, bei dem der Restaurationsrohkörper (1, 10) mit einem ein Temperatur-Zeitprofil aufweisenden Sinterverfahren gesintert wird, **dadurch gekennzeichnet, dass** das Sinteradditiv (3) nach einem der Ansprüche 1 bis 7 ausgestaltet ist.

13. Verfahren nach Anspruch 12 **dadurch gekennzeichnet, dass** das Temperatur-Zeitprofil mindestens ein Temperaturplateau mit einer Plateautemperatur und einer Plateauzeitspanne aufweist, wobei die Plateautemperatur gleich oder größer als eine für eine Gefügeumwandlung in von dem Sinteradditiv infiltrierten Infiltrationsbereichen (6) des Restaurationsrohkörpers (1, 10) erforderlichen Umwandlungstemperatur ist und die Plateauzeitspanne größer als eine für einen Beginn der Gefügeumwandlung erforderliche Umwandlungszeit ist.

14. Verfahren nach einem der Ansprüche 12 bis 13, **dadurch gekennzeichnet, dass** das Sinteradditiv (3) auf mindestens einer Kante des Restaurationsrohkörpers (1, 10) aufgetragen wird.

15. Restaurationskörperfinishingkit zur Beeinflussung der Farbanmutung eines Restaurationskörpers (16), insbesondere eines Zirkoniumrestaurationskörpers, in einem gesinterten Zustand, aufweisend einen für eine Sinterung vorgesehenen Restaurationsrohkörper (1, 10), ein Sinteradditiv (3) nach einem der Ansprüche 1 bis 7 und ein Applikationsmittel (5) zur Applikation des Sinteradditivs (3) auf dem Restaurationsrohkörper (1, 10).
